# EUROPEAN PATENT SPECIFICATION

(11) **EP 4 021 379 B1**
(45) Date of publication and mention of the grant of the patent: **18.06.2025**
(21) Application number: 20756873.4
(22) Date of filing: 19.08.2020
(51) Int. Cl.: A61K 8/06, A61K 8/36, A61K 8/34, A61K 8/58, A61K 8/86, A61K 8/891, A61Q 5/04

(54) **COMPOSITION, PROCESS AND KIT FOR SEMI-PERMANENT STRAIGHTENING AND CURLING OF KERATIN FIBERS**
ZUSAMMENSETZUNG, VERFAHREN UND KIT ZUM SEMIPERMANENTEN STRECKEN UND KRÄUSELN VON KERATINFASERN
COMPOSITION, PROCÉDÉ ET KIT DE LISSAGE ET DE BOUCLAGE DE FIBRES KÉRATINIQUES SEMI-PERMANENT

(30) Priority: 29.08.2019 EP 19194322
(43) Date of publication of application: 06.07.2022
(73) Proprietor: Kao Corporation, Chuo-ku Tokyo 103-8210 (JP)
(72) Inventor: MARUYAMA, Aguri, Tokyo, 131--0044 (JP); NIWANO, Yu, 64297 Darmstadt (DE); BAAR, Moritz, 64297 Darmstadt (DE)
(74) Representative: Hoffmann Eitle
(86) International application number: PCT/EP2020/073188
(87) International publication number: WO 2021/037629

(56) References cited:
- WO-A1-2015/036051
- WO-A1-2015/036052

## Description

### Field of the invention

The present invention is directed to a composition, process, and kit for semi-permanent straightening and curling of keratin fibers.

### Background of the invention

Changing ones hair shape has always been en vogue. Already in the ancient Egyptian era long, straight hair was popular. Women who were not blessed with this kind of natural hair already used fire heated iron plates to get their hair in the desired shape. This procedure often led to severe burns of the face and hands. It was not before the early 19th century that straightening irons became more professional with the invention of a hair tong made by Marcel Grateau. Nowadays, hot tools such as straightening irons as well as blow dryers are more popular than ever before. They came into the fashion for consumers in both salon and mass market. These tools lead to satisfying straightening results as they work at high temperatures (up to 230°C for a straightening iron). When dry hair is getting in contact with the hot iron and a proper pressure is applied at the same time, the hair shape can be fixed for a certain amount of time until humidity is return hair to its original state. Unfortunately, application of such hot temperature leads to hair damage and thus a bad hair feel after their usage.

Up to now customers who had decided for a hair straightening, were bound by their decision because current techniques did not allow them to return their hair into a curly state.

Glyoxylic acid is a known ingredient for hair cosmetics which allow for long-lasting curl relaxation and volume reduction especially in combination with straightening irons at high temperatures (WO2011/104282, WO2012/010351). However, once the customer has decided for such a treatment, it is almost impossible to return to the original curly hair state.

WO2015/036051 and WO201036052 illustrate the use of glyoxylic acid in straightening processes in combination with blow-dry heat and ironing heat as well as post-straightening compositions.

WO2013/081018 discloses alkyl glyceryl ether in combination with lactic acid as a hair treatment agent. However, the present invention requires glyoxylic acid.

### Summary of the invention

Therefore, the first object of the present invention is an aqueous cosmetic composition having a pH of less than 4 comprising:
a) glyoxylic acid and/or its salt(s) and/or its hydrate(s),
b) one or more alkyl glyceryl ether and/or alkyl polyglyceryl ether, and/or their mixtures, wherein the alkyl chain is a straight or branched, saturated or unsaturated alkyl chain having a total carbon number of 8 or more,
c) one or more lipophilic compound(s),
d) one or more surfactant(s) different from group b),
wherein the composition is an emulsion.

The second object of the present invention is a process for reshaping keratin fibers, preferably human keratin fibers, more preferably human hair, comprising the steps of:
i) applying the composition as defined in the claims 1 to 10 onto keratin fibers and leaving it for a time period of 1 to 60 min,
ii) optionally rinsing-off the keratin fibers and optionally drying the keratin fibers,
iii) treating the keratin fibers with a hot tool in a temperature range from 45°C to 230°C,
iv) optionally rinsing-off the keratin fibers,
v) optionally curling the keratin fibers with a curling tool in a temperature range from 45°C to 230°C,
vi) optionally rinsing-off the keratin fibers.

The third object of the present invention is a process for reshaping keratin fibers, preferably human keratin fibers, more preferably human hair comprising the steps of:
vii) applying an aqueous composition having a pH of less than 4 and comprising one or more compound(s) according to a) as defined in claims 1 to 3,
viii) leaving the composition onto keratin fibers for a time period of 1 to 60 min,
ix) optionally rinsing-off and optionally drying the keratin fibers,
x) treating the keratin fibers with a hot tool in a temperature range of 45°C to 230°C,
xi) applying a composition comprising compounds b) to d) as defined above onto keratin fibers,
xii) leaving the composition for a time period of 1 to 60 min,
xiii) optionally treating the keratin fibers with a hot tool in a temperature range of 45°C to 230°C,

The fourth object of the present invention is a kit-of-parts comprising a composition as defined above and a hot tool.

A fifth object of the present invention is a kit-of-parts comprising a first composition as defined above in step vii) and a second composition as defined above in step xi).

### Description of figures

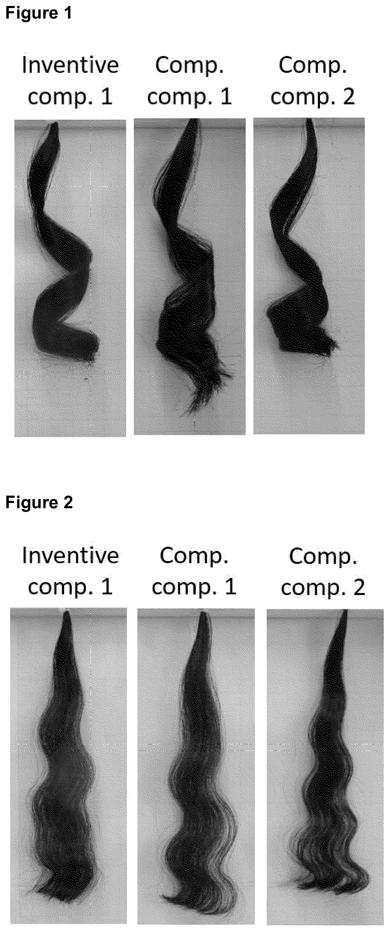

Figure 1 is a photograph of the hair streaks treated with the compositions of example 1. The photograph was taken after the curling step. As clearly observable, the hair streak treated with comparative composition 1 exhibited the weakest curl resulting from the limited restyling ability of hair treated with comparative composition 1.
Figure 2 is a photograph of the hair streaks treated with the compositions of example 1. The photograph was taken after the blow dry and brushing step. It illustrates the straightening degree after curling without employing a flat-ironing step. As clearly observable, the hair treated with comparative composition 2 exhibited greater waviness resulting from the limited straightness of hair treated with comparative composition 2.

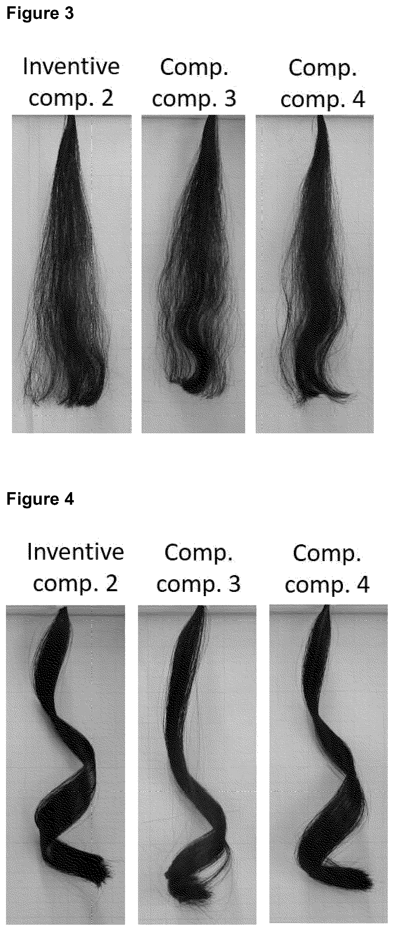

Figure 3 is a photograph of the hair streaks treated with the compositions of example 2. The photograph was taken after the brushing and blow-drying step. As clearly observable, the hair streaks treated with comparative compositions 3 and 4 exhibited more curling, especially at the tip of the streaks, whereas the inventive composition 2 delivered the straightest hair streak.
Figure 4 is a photograph of the hair streaks treated with the compositions of example 2. The photograph was taken after the curl-ironing step. As clearly observable, the hair streaks treated with inventive composition 2 and comparative composition 4 exhibited stronger curling, whereas the comparative composition 3 delivered the weakest curl.

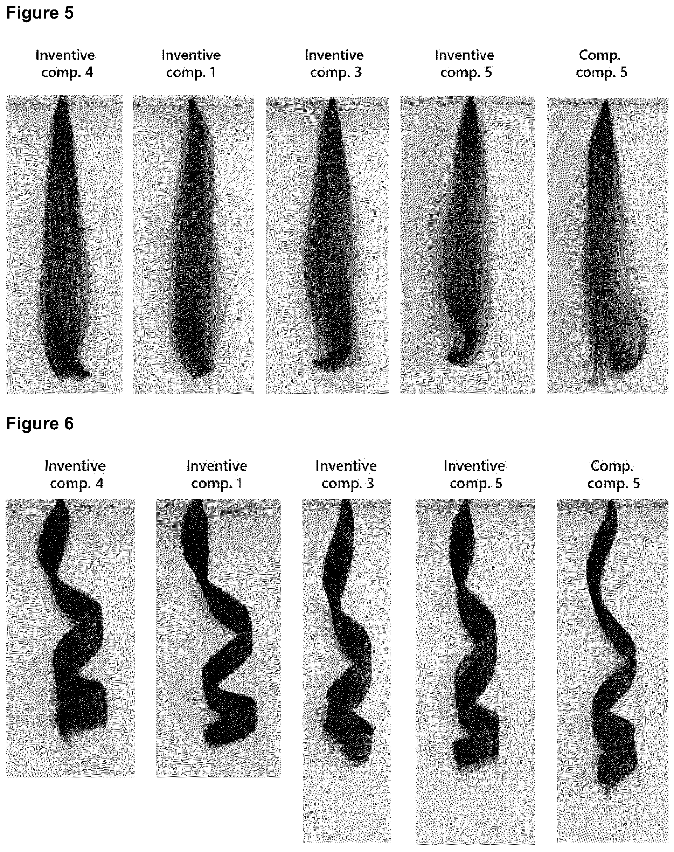

Figure 5 is a photograph of the hair streaks treated with the compositions of example 3. The photograph was taken after the brushing and blow-drying step. As clearly observable, the hair streak treated with comparative composition 5 was less defined and had a fuzzy end, whereas the inventive compositions delivered the most defined hair streaks.
Figure 6 is a photograph of the hair streaks treated with the compositions of example 3. The photograph was taken after the curl-ironing step. As clearly observable, the hair streaks treated with inventive compositions had much stronger curling compared to the comparative composition 5. The latter had the weakest curl of the set.

### Detailed description of the invention

The present invention addresses the above-mentioned problem of the prior art and allows for a transformation from curly to straight, and if desired, back to original curly keratin fibers. Additionally, straightness of keratin fibers is improved for the transformation from curly to straight with or without prior curling step.

The present invention is directed to an aqueous cosmetic composition having a pH of less than 4 comprising:
a) glyoxylic acid and/or its salt(s) and/or its hydrate(s),
b) one or more alkyl glyceryl ether and/or alkyl polyglyceryl ether, and/or their mixtures, wherein the alkyl chain is a straight or branched, saturated or unsaturated alkyl chain having a total carbon number of 8 or more,
c) one or more lipophilic compound(s),
d) one or more surfactant(s) different from group b),
wherein the composition is an emulsion.

Without being bound by any theory, applicant has found out that compounds according to b) allow hair to undergo a thermoplastic transition. It is believed that these compounds act as plasticizers to hair. Thus, the inventive composition allows the customers to move their hairstyle from curly to straight, and then back from straight to curly hair.

It is preferred from the viewpoint of cosmetic acceptance and safety that the pH of the composition is in the range of 0.5 to 3.5, preferably in the range of 1 to 3, more preferably in the range of 1.5 to 2.5.

It is further preferred from the viewpoint of cosmetic acceptance and customer usability that the composition of the present invention is an oil-in-water emulsion.

### Compounds according to a)

The composition of the present invention comprises glyoxylic acid and/or its salt(s) and/or its hydrate(s) as compounds according to a).

Glyoxylic acid may be used for the purpose of the present invention in its free acid form and its hydrate thereof. The hydrate is formed by providing an aqueous solution of glyoxylic acid and without any further pH adjustment, glyoxylic acid is almost quantitatively present as its hydrate. Additionally, the hydrate may condense to dimers.

A salt of glyoxylic acid may also be used. A salt is inevitably formed by adjusting the pH of the composition with any known base. Suitable salts are alkali metal salts such as sodium or potassium, alkaline earth metal salts such as magnesium or calcium, or ammonium salts.

It is preferred from the viewpoint of straightening efficiency that the concentration of compound(s) according to a) is 0.1% by weight or more, more preferably 0.2% by weight or more, further more preferably 0.5% by weight or more, still further more preferably 1% by weight or more, calculated to the total weight of the composition.

It is preferred from the viewpoint of straightening efficiency and cosmetic safety that the concentration of compound(s) according to a) is 25% by weight or less, more preferably 20% by weight or less, further more preferably 15% by weight or less, still further more preferably 12% by weight or less, calculated to the total weight of the composition.

For attaining the above-mentioned effect, it is preferred that the concentration of compound(s) according to a) is in the range of 0.1% to 25% by weight, preferably in the range of 0.2% to 20% by weight, more preferably in the range of 0.5% to 15% by weight, further more preferably 1% to 12% by weight, calculated to the total weight of the composition.

### Compounds according to b)

The composition of the present invention comprises one or more alkyl glyceryl ether and/or alkyl polyglyceryl ether, and/or their mixtures, wherein the alkyl chain is a straight or branched, saturated or unsaturated alkyl chain having a total carbon number of 8 or more, as compounds according to b).

It is preferred from the viewpoint of oil solubility and emulsification that the compounds according to b) have an HLB value in the range of 2 to 7. This allows for a preparation of a co-emulsion with the compounds according to c) and d).

However, in case the compounds according to b) have other HLB values, they are dispersable in the aqueous phase of the composition according to the present invention.

From the viewpoint of emulsification, the alkyl chain of compounds according to b) is a straight or branched, saturated or unsaturated alkyl chain having a total carbon number of 8 or more.

It is preferred from the viewpoint of conferring thermoplastic properties to keratin fibers that the alkyl chain of compounds according to b) is a straight or branched, saturated or unsaturated alkyl chain having a total carbon number of 9 or more.

It is preferred from the viewpoint of emulsification that the alkyl chain of compounds according to b) is a straight or branched, saturated or unsaturated alkyl chain having a total carbon number of 18 or less.

For attaining the above-mentioned effect, it is preferred that the alkyl chain of compounds according to b) is a straight or branched, saturated or unsaturated alkyl chain having a total carbon number in the range of 8 to 18, more preferably in the range of 9 to 18.

Suitable examples for compounds according to b) are isostearyl glyceryl ether, stearyl glyceryl ether, isodecyl glyceryl ether, 2-ethylhexyl glyceryl ether, and cetyl glyceryl ether as well as alkyl polyglyceryl ether as disclosed, for example, in EP2003110.

It is preferred from the viewpoint of commercial availability and conferring thermoplastic properties to keratin fibers that one or more compound(s) according to b) is isostearyl glyceryl ether.

It is further preferred from the viewpoint of hair transformation that the total concentration of one or more compound(s) according to b) is 0.1% by weight or more, more preferably 0.25% by weight or more, still more preferably 0.5% by weight or more, calculated to the total weight of the composition.

It is further preferred from the viewpoint of hair transformation that the total concentration of one or more compound(s) according to b) is 10% by weight or less, more preferably 8% by weight or less, still more preferably 5% by weight or less, calculated to the total weight of the composition.

For attaining the above-mentioned effects, the total concentration of one or more compound(s) according to b) is in the range of 0.1% to 10% by weight, preferably 0.25% to 8% by weight, more preferably 0.5% to 5% by weight, calculated to the total weight of the composition.

### Compounds according to c)

The composition of the present invention comprises one or more lipophilic compound(s).

Lipophilic compounds according to c ) are immiscible with water at 25°C under atmospheric pressure. However, these compounds are emulsifiyable with the help of compounds according to d) to form an emulsion.

Suitable compounds according to c) are from fatty alcohols having a linear or branched, saturated or unsaturated alkyl chain with a total carbon number in the range of C8 to C22 which may be optionally substituted with OH, fatty acid esters having esterified an linear or branched alcohol having an alkyl chain length of C3 to C22 with a linear or branched fatty acid having an alkyl chain length of C12 to C22, petrolatum-based compound(s), organopolysiloxane(s), vegetable oils, and/or their mixtures.

Suitable fatty alcohols having a linear or branched, saturated or unsaturated alkyl chain with a total carbon number in the range of C8 to C22 which may be optionally substituted with OH, are, for example, octyl alcohol, decyl alcohol, lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures.

Suitable fatty acid esters having esterified an linear or branched alcohol having an alkyl chain length of C3 to C22 with a linear or branched fatty acid having an alkyl chain length of C12 to C22, are, for example, octyl palmitate, isocetyl palmitate, isopropyl palmitate, octyl stearate, oleyl oleate, and myristyl myristate, as well as their mixtures.

Suitable petrolatum-based compound(s) are, for example, mineral oil, polydecenes, Vaseline, paraffinum perliquidum, and paraffinum subliquidum, and/or their mixtures.

Suitable organopolysiloxanes are in particular the ones which are not soluble in water, as, for example, such as dimethicones with various viscosities and molecular weights and dimethiconols, aminated silicones with primary, secondary, tertiary or quaternary ammonium groups such as amodimethicone, polysilicone 9, and quaternium 80, cyclic silicones such as cyclomethicones, arylated silicones such as phenyl trimethicone.

Suitable vegetable oils are olive oil, almond oil, avocado oil, wheatgerm oil, and castor oil.

It is preferred from the viewpoint of emulsion stability that the total concentration of compound(s) according to c) is 0.5% by weight or more, more preferably 1% by weight or more, further more preferably 2% by weight or more, calculated to the total weight of the composition.

It is preferred from the viewpoint of emulsion stability and emulsion type that the total concentration of compound(s) according to c) is 25% by weight or less, more preferably 20% by weight or less, further more preferably 15% by weight or less, calculated to the total weight of the composition.

The composition according to any of the preceding claims characterized in that the total concentration of compound(s) according to c) is in the range of 0.5% to 25% by weight, preferably 1% to 20% by weight, more preferably 2% to 15% by weight, calculated to the total weight of the composition.

### Compounds according to d)

The composition of the present invention comprises one or more surfactant(s) as compound(s) according to d) which is different from group b).

Compounds according to d) ensure the stability of the emulsion.

It is preferred from the viewpoint of emulsion stability that one or more compound(s) according to d) is/are selected from non-ionic surfactant(s), cationic surfactant(s), anionic surfactant(s), amphoteric/zwitterionic surfactant(s), and/or their mixtures, preferably it is selected from non-ionic and/or cationic surfactant(s).

Suitable non-ionic surfactant preferably have an HLB value in the range of 7 to 14 from the viewpoint of stabilizing the emulsion.

Suitable non-ionic surfactants are, for examples, fatty alcohol ethoxylates, propylene glycol ethers of fatty alcohols, polyethylene glycol or polypropylene glycol esters of fatty acids, ethoxylated or propoxylated triglycerides, alkyl polyglycosides, and/or their mixtures.

Suitable cationic surfactants are, for example, quaternary ammonium salts having an alkyl chain length in the range of C12 to C22. Suitable examples are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate, and/or their mixtures.

Suitable anionic surfactants are, for example, alkyl sulfate or ethoxylated alkyl ether sulfate surfactants with an alkyl chain length of C₁₀ to C₂₂, and/or their mixtures.

Suitable surfactants are laureth sulfates, coceth sulfate, pareth sulfate, capryleth sulphate, myreth sulfate, oleth sulfate, deceth sulfate, trideceth sulfate, coco sulphate, C₁₀-C₁₆ alkyl sulphate, C₁₁-C₁₅ alkyl sulphate, C₁₂-C₁₈ alkyl sulphate, C₁₂-C₁₅ alkyl sulphate, C₁₂-C₁₆ alkyl sulphate, C₁₂-C₁₃ alkyl sulfate, lauryl sulphate, myristyl sulphate, palm kernel sulphate, cetearyl sulfate, cetyl sulphate, decyl sulphate, oleyl sulphate, behenyl sulphate and/or their salts, and/or their mixtures. All of the aforementioned anionic surfactants may or may not be ethoxylated at various degrees.

Cations for anionic surfactants may be selected from sodium, potassium, magnesium and/or ammonium.

Suitable amphoteric/zwitterionic surfactant(s) are, for example, various known betaines such as alkyl betaines, fatty acid amidoalkyl betaines and sulfobetaines such as lauryl hydroxysulfobetaine; long-chain alkyl amino acids, such as cocoaminoacetate, cocoaminopropionate, sodium cocoamphopropionate and - acetate are suitable, and/or their mixtures.

The most preferred surfactants are cationic surfactants, in particular cetyl trimethyl ammonium chloride, from the viewpoint of emulsion stability.

It is preferred from the viewpoint of emulsion stability that the total concentration of compound(s) according to d) is 0.2% by weight or more, more preferably 0.5% by weight or more, still more preferably 1% by weight or more, calculated to the total weight of the composition.

It is preferred from the viewpoint of emulsion stability that the total concentration of compound(s) according to d) is 10% by weight or less, more preferably 5% by weight or less, still more preferably 3% by weight or less, calculated to the total weight of the composition.

For attaining the above-mentioned effects, it is preferred that the total concentration of compound(s) according to d) is in the range of 0.2% to 10% by weight, more preferably 0.5% to 5% by weight, still more preferably 1% to 3% by weight, calculated to the total weight of the composition.

### One-step treatment method

The present invention is also directed to a process for reshaping keratin fibers, preferably human keratin fibers, more preferably human hair, comprising the steps of:
i) applying the composition as defined in the claims 1 to 10 onto keratin fibers and leaving it for a time period of 1 to 60 min,
ii) optionally rinsing-off the keratin fibers and optionally drying the keratin fibers,
iii) treating the keratin fibers with a hot tool in a temperature range from 45°C to 230°C,
iv) optionally rinsing-off the keratin fibers,
v) optionally curling the keratin fibers with a curling tool in a temperature range from 45°C to 230°C,
vi) optionally rinsing-off the keratin fibers.

Preferably, from the viewpoint of straightening, the time range of step i) is in the range of 5 to 45 min, more preferably in the range of 10 to 30 min.

For step iii), it is preferred from the viewpoint of straightening that the hot tool is a flat-iron for straightening having a surface temperature in the range of 90°C to 230°C, more preferably 130°C to 210°C. Alternatively, from the viewpoint of consumer applicability, a blow dryer and comb may be used having an air temperature in the range of 45°C to 90°C.

For the curling step v) it is preferred to use a curling iron or a hot comb with a further preferred temperature range from 90°C to 210°C, from the viewpoint of curling efficiency and hair damage.

### Two-step treatment method

The present invention also is directed to a process for reshaping keratin fibers, preferably human keratin fibers, more preferably human hair comprising the steps of:
vii) applying an aqueous composition having a pH of less than 4 and comprising one or more compound(s) according to a) as defined above,
viii) leaving the composition onto keratin fibers for a time period of 1 to 60 min,
ix) optionally rinsing-off and optionally drying the keratin fibers,
x) treating the keratin fibers with a hot tool in a temperature range of 45°C to 230°C,
xi) applying a composition comprising compounds b) to d) as defined above onto keratin fibers,
xii) leaving the composition for a time period of 1 to 60 min,
xiii) optionally treating the keratin fibers with a hot tool in a temperature range of 45°C to 230°C,

As further optional steps, the process of above may comprise:
xv) optionally curling the keratin fibers with a curling tool in a temperature range from 45°C to 230°C,
xvi) optionally rinsing-off the keratin fibers.

The two-step process is an alternative for the one-step process and yield equally favourable results.

Preferably, from the viewpoint of straightening, the time range of steps vii) and/or xii) is in the range of 5 to 45 min, more preferably in the range of 10 to 30 min.

For steps x) and/or xiii), it is preferred from the viewpoint of straightening that a flat-iron is used for straightening having a surface temperature in the range of 90°C to 230°C, more preferably 130°C to 210°C. Alternatively, from the viewpoint of consumer applicability, a blow dryer and comb may be used having an air temperature in the range of 45°C to 90°C.

For the curling step xv) it is preferred to use a curling iron or a hot comb with a further preferred temperature range from 90°C to 210°C, from the viewpoint of curling efficiency and hair damage.

### Kit-of-parts

The present invention is further directed to a kit-of-parts comprising the inventive composition as defined above and a hot tool. Suitable hot tools are blow dryers, hot combs, curling irons, and/or straightening irons.

The invention is further directed to a kit-of-parts comprising a first composition as defined above for step vii) and a second composition as defined above in step xi). Optionally, the kit may further comprise a hot tool. Suitable hot tools are blow dryers, hot combs, curling irons, and/or straightening irons.

The following examples are to illustrate the present invention, but not to limit it.

### EXAMPLES

### Example 1

The following compositions were prepared by adding compound b), c), and d) into water and heating the mixture under stirring above 70°C. After cooling, compound a) or lactic acid as well as NaOH were added.

| **Compound** | **Ingredients** | **Inventive composition 1** | **Comparative composition 1** | **Comparative composition 2** |
|---|---|---|---|---|
| **a)** | Glyoxylic acid | 0.8 | 0.8 | - |
| **-** | Lactic acid | - | - | 0.8 |
| **b)** | Isostearyl glyceryl ether | 3.5 | - | 3.5 |
| **c)** | Cetearyl alcohol | 3.0 | 3.0 | 3.0 |
| **d)** | Ceteareth-30 | 1.5 | 1.5 | 1.5 |
| | NaOH ad pH | Ad pH 2.2 | | |
| | Water | Ad 100.0 | | |

Human hair streaks (Caucasian hair, 2 g per bundle, 21 cm long) were purchased from Fischbach + Miller Haar, Laupheim, Germany. The hair streaks were shampooed with a commercially available shampoo under the brand name Goldwell Dualsenses Deep Cleansing Shampoo. 1 g of each of the compositions from above were then applied on the hair streaks and the compositions were left for 3 min onto the hair streaks. Then the hair streaks were rinsed for 1 min with lukewarm water and blow dried. As a next step, the hair streaks were treated with 3 strokes of a flat iron at 160°C iron surface temperature. At this point, straightness of the hair streaks was evaluated.

Then the hair streaks were treated with 1 pass of a curl iron at 180°C iron surface temperature for 10 s per hair streak. At this point, curling of the hair streaks was evaluated (see Fig. 1).

As a last step, the hair streaks were shampooed with the commercially available shampoo of above, combed, and blow dried. At this point, straightness of the hair streaks was evaluated again (see Fig. 2).

The following results were obtained as displayed in the table below and illustrated in Figures 1 and 2:

| **Process step** | **Inventive composition 1** | **Comparative composition 1** | **Comparative composition 2** |
|---|---|---|---|
| **Straightening after flat-ironing** | Very straight | Very straight | Very straight |
| **Curling after curl-ironing (****Fig. 1****)** | Strong curl | Weak curl | Strong curl |
| **Straightening after shampoo and blow drying (****Fig. 2****)** | Slightly wavy | Slightly wavy | Wavy |

As a result from comparative testing above, the inventive composition delivered best restyling ability from straight to curly and back to straight hair. None of the two comparative compositions exhibited the same performance.

### Example 2

The following compositions were prepared by adding compound b), c), and d) or their equivalents for comparative purposes into water and heating the mixture under stirring above 70°C. After cooling, compound a) or lactic acid as well as NaOH were added.

| **Compound** | **Ingredients** | **Inventive composition 2** | **Comparative composition 3** | **Comparative composition 4** |
|---|---|---|---|---|
| **a)** | Glyoxylic acid | 0.8 | 0.8 | - |
| | Lactic acid | - | - | 0.8 |
| **b)** | Isostearyl glyceryl ether | 3.5 | - | 3.5 |
| **c)** | Cetearyl alcohol | 3.0 | 3.0 | 3.0 |
| **d)** | Cetrimonium chloride | 1.5 | 1.5 | 1.5 |
| | NaOH ad pH | Ad pH 2.2 | | |
| | Water | Ad 100.0 | | |

Human hair streaks (Caucasian hair, 2 g per bundle, 21 cm long) were purchased from Fischbach + Miller Haar, Laupheim, Germany. The hair streaks were shampooed with a commercially available shampoo under the brand name Goldwell Dualsenses Deep Cleansing Shampoo. 1 g of each of the compositions from above were then applied on the hair streaks and the compositions were left for 3 min onto the hair streaks. Then the hair streaks were rinsed for 1 min with lukewarm water and blow dried. The aforementioned application, rinsing, and blow-drying steps were repeated 2 more times, except for the last blow-drying step which was replaced by air-drying. As a next step, the hair streaks were brushed and blow-dried with hot air having a temperature of 60°C. At this point, straightness of the hair streaks was evaluated (see Fig. 3).

Then the hair streaks were treated with 1 pass of a curl iron at 130°C iron surface temperature for 10 s each. At this point, curling of the hair streaks was evaluated (see Fig. 4).

The following results were obtained as displayed in the table below and illustrated in Figures 3 and 4:

| **Process step** | **Inventive composition 2** | **Comparative composition 3** | **Comparative composition 4** |
|---|---|---|---|
| **Straightening after brushing and blow-drying (****Fig. 3****)** | Straight | Little end curl | Medium end curl |
| **Curling after curl-ironing (****Fig. 4****)** | Strong curl | Weak curl | Strong curl |

As a result from comparative testing above, the inventive composition delivered best restyling ability from straight to curly hair. None of the two comparative compositions exhibited the same performance.

### EXAMPLE 3

The following compositions were prepared by adding compound b), c), and d) or their equivalents for comparative purposes into water and heating the mixture under stirring above 70°C. After cooling, compound a) or lactic acid as well as NaOH were added.

| | **Ingredients** | **Inventive comp. 1** | **Inventive comp. 3** | **Inventive comp. 4** | **Inventive comp. 5** | **Comparative comp. 5** |
|---|---|---|---|---|---|---|
| **a)** | Glyoxylic acid | 0.8 | 0.8 | 0.8 | 0.8 | 0.8 |
| **-** | Lactic acid | - | | | | - |
| **b)** | Isostearyl glyceryl ether | 3.5 | | 10.0 | | - |
| | Cetyl glyceryl ether | - | 3.5 | - | - | - |
| | Hexyldecyl polyglyceryl ether | - | - | - | 3.5 | - |
| - | PEG-18 glyceryl oleate/cocoate | - | - | - | - | 3.5 |
| **c)** | Cetearyl alcohol | 3.0 | 3.0 | 3.0 | 3.0 | 3.0 |
| **d)** | Ceteareth-30 | 1.5 | 1.5 | 1.5 | 1.5 | 1.5 |
| - | NaOH ad pH | Ad pH 2.2 | | | | |
| | Water | Ad 100.0 | | | | |

For the compositions above, the same experimental protocol as explained for example 2 was used.

Straightness of the hair streaks was evaluated (see Fig. 5).

Then the hair streaks were treated with 1 pass of a curl iron at 130°C iron surface temperature for 10 s each. At this point, curling of the hair streaks was evaluated (see Fig. 6).

The following results were obtained as displayed in the table below and illustrated in Figures 5 and 6:

| **Process step** | **Inv. comp. 1** | **Inv. Comp. 3** | **Inv. Comp. 4** | **Inv. Comp. 5** | **Comparative comp. 5** |
|---|---|---|---|---|---|
| **Straightening after brushing and blow-drying (****Fig. 5****)** | Very straight, medium bundling, no end-curl | Straight, strong bundling, small end-curl | Very straight, strong bundling, no end-curl | Very straight, medium bundling, small end-curl | Very straight, low bundling, small, fuzzy end-curl |
| **Curling after curl-ironing (****Fig. 6****)** | Strong curl, defined end-curl | Medium curl, slightly fuzzy end-curl | Strong curl, defined end-curl | Medium curl, slightly fuzzy end-curl | Low curl, slightly fuzzy end-curl |

As a result from comparative testing above, the inventive compositions delivered best restyling ability from straight to curly hair. The comparative composition particularly suffered from fuzzy straightness with low bundling, and lower curling performance.

The following examples are within the scope of the present invention.

### EXAMPLE 4

| | **% by weight** |
|---|---|
| Glyoxylic acid | 6.0 |
| Isostearyl glyceryl ether | 2.0 |
| Stearyl alcohol | 5.0 |
| Behentrimonium chloride | 0.5 |
| Behenamidopropyl dimethylamine | 0.5 |
| Ceteareth-33 | 0.1 |
| Glycerine | 0.25 |
| Amodimethicone | 0.5 |
| Xanthan gum | 1.0 |
| NaOH | q.s. ad pH 3.5 |
| Water | ad 100.0 |

### EXAMPLE 5

| | **% by weight** |
|---|---|
| Glyoxylic acid | 2.0 |
| Isodecyl glyceryl ether | 3.0 |
| Cetearyl alcohol | 5.0 |
| Isopropyl myristate | 2.0 |
| PEG-40 hydrogenated castor oil | 3.0 |
| Benzyl alcohol | 0.5 |
| Dimethicone | 0.25 |
| Polyquaternium 10 | 0.5 |
| Hydroxyethyl cellulose | 1.5 |
| NaOH | q.s. ad pH 2.5 |
| Water | ad 100.0 |

### EXAMPLE 6

| | **% by weight** |
|---|---|
| Glyoxylic acid | 10.0 |
| Isostearyl glyceryl ether | 1.0 |
| Cetearyl alcohol | 5.0 |
| Behenamidopropyl dimethylamine | 0.75 |
| Isopropyl Palmitate | 2.0 |
| Dipropylene Glycol | 2.0 |
| Panthenol | 0.5 |
| Divinyldimethicone/Dimethicone Copolymer | 0.75 |
| Dehydroxanthan Gum | 1.0 |
| Phenoxyethanol | 0.5 |
| NaOH | q.s. ad pH 2.0 |
| Water | ad 100.0 |

## Claims

1. An aqueous cosmetic composition having a pH of less than 4 comprising:
a) glyoxylic acid and/or its salt(s) and/or its hydrate(s),
b) one or more alkyl glyceryl ether and/or alkyl polyglyceryl ether, and/or their mixtures, wherein the alkyl chain is a straight or branched, saturated or unsaturated alkyl chain having a total carbon number of 8 or more,
c) one or more lipophilic compound(s),
d) one or more surfactant(s) different from group b),
wherein the composition is an emulsion.

2. The composition according to claim 1 **characterized in that** the pH of the composition is in the range of 0.5 to 3.5, preferably in the range of 1 to 3, more preferably in the range of 1.5 to 2.5.

3. The composition according to claims 1 and/or 2 **characterized in that** the concentration of compound(s) according to a) is in the range of 0.1 to 25% by weight, preferably in the range of 0.2% to 20% by weight, more preferably in the range of 0.5% to 15% by weight, further more preferably 1% to 12% by weight, calculated to the total weight of the composition.

4. The composition according to any of the preceding claims **characterized in that** the alkyl chain of compounds according to b) is a straight or branched, saturated or unsaturated alkyl chain having a total carbon number of 9 or more.

5. The composition according to any of the preceding claims **characterized in that** one or more compound(s) according to b) is isostearyl glyceryl ether.

6. The composition according to any of the preceding claims **characterized in that** the total concentration of one or more compound(s) according to b) is in the range of 0.1% to 10% by weight, preferably 0.25% to 8% by weight, more preferably 0.5% to 5% by weight, calculated to the total weight of the composition.

7. The composition according to any of the preceding claims **characterized in that** compound(s) according to c) are selected from fatty alcohols having a linear or branched, saturated or unsaturated alkyl chain with a total carbon number in the range of C8 to C22 which may be optionally substituted with OH, fatty acid esters having esterified an linear or branched alcohol having an alkyl chain length of C3 to C22 with a linear or branched fatty acid having an alkyl chain length of C12 to C22, petrolatum-based compound(s), organopolysiloxane(s), vegetable oils, and/or their mixtures.

8. The composition according to any of the preceding claims **characterized in that** the total concentration of compound(s) according to c) is in the range of 0.5% to 25% by weight, preferably 1% to 20% by weight, more preferably 2% to 15% by weight, calculated to the total weight of the composition.

9. The composition according to any of the preceding claims **characterized in that** one or more compound(s) according to d) is/are selected from non-ionic surfactant(s), cationic surfactant(s), anionic surfactant(s), amphoteric/zwitterionic surfactant(s), and/or their mixtures, preferably it is selected from non-ionic and/or cationic surfactant(s).

10. The composition according to any of the preceding claims **characterized in that** the total concentration of compound(s) according to d) is in the range of 0.2% to 10% by weight, preferably 0.5% to 5% by weight, more preferably 1% to 3% by weight, calculated to the total weight of the composition.

11. A process for reshaping keratin fibers, preferably human keratin fibers, more preferably human hair, comprising the steps of:
i) applying the composition as defined in the claims 1 to 10 onto keratin fibers and leaving it for a time period of 1 to 60 min,
ii) optionally rinsing-off the keratin fibers and optionally drying the keratin fibers,
iii) treating the keratin fibers with a hot tool in a temperature range from 45°C to 230°C,
iv) optionally rinsing-off the keratin fibers,
v) optionally curling the keratin fibers with a curling tool in a temperature range from 45°C to 230°C,
vi) optionally rinsing-off the keratin fibers.

12. A process for reshaping keratin fibers, preferably human keratin fibers, more preferably human hair comprising the steps of:
vii) applying an aqueous composition having a pH of less than 4 and comprising one or more compound(s) according to a) as defined in claims 1 to 10,
viii) leaving the composition onto keratin fibers for a time period of 1 to 60 min,
ix) optionally rinsing-off and optionally drying the keratin fibers,
x) treating the keratin fibers with a hot tool in a temperature range of 45°C to 230°C,
xi) applying a composition comprising compounds b) to d) as defined in the claims 1 to 10 onto keratin fibers,
xii) leaving the composition for a time period of 1 to 60 min,
xiii) optionally treating the keratin fibers with a hot tool in a temperature range of 45°C to 230°C,
xiv) optionally rising-off the keratin fibers,

13. The process according to claim 11 and/or 12 **characterized in that** the straightening step(s) are carried out by using a straightening iron having a temperature in the range of 90°C to 230°C or by using a blow dryer and a comb in the temperature range of 45°C to 90°C.

14. A kit-of-parts comprising a composition as defined in claims 1 to 10 and a hot tool.

15. A kit-of-parts comprising a first composition as defined in claim 12 step vii) and a second composition as defined in claim 12 step xi).

## Patentansprüche

1. Wässrige Kosmetikzusammensetzung mit einem pH-Wert von weniger als 4, umfassend:
a) Glyoxylsäure und/oder Salz(e) und/oder Hydrat(e) davon,
b) einen oder mehrere Alkylglycerylether und/oder Alkylpolyglycerylether und/oder Mischungen davon, wobei die Alkylkette eine lineare oder verzweigte, gesättigte oder ungesättigte Alkylkette mit einer Gesamtkohlenstoffzahl von 8 oder mehr ist,
c) eine oder mehr lipophile Verbindung(en),
d) ein oder mehr Tensid(e), verschieden von Gruppe b),
wobei die Zusammensetzung eine Emulsion ist.

2. Zusammensetzung gemäß Anspruch 1, **dadurch gekennzeichnet, dass** der pH-Wert der Zusammensetzung im Bereich von 0,5 bis 3,5, bevorzugt im Bereich von 1 bis 3, mehr bevorzugt im Bereich von 1,5 bis 2,5, liegt.

3. Zusammensetzung gemäß den Ansprüchen 1 und/oder 2, **dadurch gekennzeichnet, dass** die Konzentration der Verbindung(en) gemäß a) im Bereich von 0,1 bis 25 Gew.-%, bevorzugt im Bereich von 0,2 bis 20 Gew.-%, mehr bevorzugt im Bereich von 0,5 bis 15 Gew.-%, noch mehr bevorzugt 1 bis 12 Gew.-%, berechnet auf das Gesamtgewicht der Zusammensetzung, liegt.

4. Zusammensetzung gemäß einem der vorstehenden Ansprüche, **dadurch gekennzeichnet, dass** die Alkylkette der Verbindungen gemäß b) eine lineare oder verzweigte, gesättigte oder ungesättigte Alkylkette mit einer Gesamtkohlenstoffzahl von 9 oder mehr ist.

5. Zusammensetzung gemäß einem der vorstehenden Ansprüche, **dadurch gekennzeichnet, dass** eine oder mehr Verbindung(en) gemäß b) Isostearylglycerylether sind.

6. Zusammensetzung gemäß einem der vorstehenden Ansprüche, **dadurch gekennzeichnet, dass** die Gesamtkonzentration der einen oder mehr Verbindung(en) gemäß b) im Bereich von 0,1 bis 10 Gew.-%, bevorzugt 0,25 bis 8 Gew.-%, mehr bevorzugt 0,5 bis 5 Gew.-%, berechnet auf das Gesamtgewicht der Zusammensetzung, liegt.

7. Zusammensetzung gemäß einem der vorstehenden Ansprüche, **dadurch gekennzeichnet, dass** Verbindung(en) gemäß c) aus Fettalkoholen mit einer linearen oder verzweigten, gesättigten oder ungesättigten Alkylkette mit einer Gesamtkohlenstoffzahl im Bereich von C8 bis C22, die optional mit OH substituiert sein kann, Fettsäureestern, die einen linearen oder verzweigten Alkohol mit einer Alkylkettenlänge von C3 bis C22, der mit einer linearen oder verzweigten Fettsäure mit einer Alkylkettenlänge von C12 bis C22 verestert ist, aufweisen, Verbindung(en) auf Petrolatum-Basis, Organopolysiloxan(en), Pflanzenölen und/oder Mischungen davon ausgewählt sind.

8. Zusammensetzung gemäß einem der vorstehenden Ansprüche, **dadurch gekennzeichnet, dass** die Gesamtkonzentration der Verbindung(en) gemäß c) im Bereich von 0,5 bis 25 Gew.-%, bevorzugt 1 bis 20 Gew.-%, mehr bevorzugt 2 bis 15 Gew.-%, berechnet auf das Gesamtgewicht der Zusammensetzung, liegt.

9. Zusammensetzung gemäß einem der vorstehenden Ansprüche, **dadurch gekennzeichnet, dass** eine oder mehr Verbindung(en) gemäß d) aus nichtionischem(n) Tensid(en), kationischem(n) Tensid(en), anionischem(n) Tensid(en), amphoterem(n)/zwitterionischem(n) Tensid(en) und/oder Mischungen davon ausgewählt ist/sind, bevorzugt aus nichtionischem(n) und/oder kationischem(n) Tensid(en) ausgewählt ist.

10. Zusammensetzung gemäß einem der vorstehenden Ansprüche, **dadurch gekennzeichnet, dass** die Gesamtkonzentration der Verbindung(en) gemäß d) im Bereich von 0,2 bis 10 Gew.-%, bevorzugt 0,5 bis 5 Gew.-%, mehr bevorzugt 1 bis 3 Gew.-%, berechnet auf das Gesamtgewicht der Zusammensetzung, liegt.

11. Verfahren zum Umformen von Keratinfasern, bevorzugt menschlichen Keratinfasern, mehr bevorzugt menschlichem Haar, umfassend die Schritte:
i) das Auftragen der Zusammensetzung, wie in den Ansprüchen 1 bis 10 definiert, auf Keratinfasern und das Belassen für einen Zeitraum von 1 bis 60 min,
ii) optional das Abspülen der Keratinfasern und optional das Trocknen der Keratinfasern,
iii) das Behandeln der Keratinfasern mit einem heißen Werkzeug in einem Temperaturbereich von 45°C bis 230°C,
iv) optional das Abspülen der Keratinfasern,
v) optional das Kräuseln der Keratinfasern mit einem Kräuselwerkzeug in einem Temperaturbereich von 45°C bis 230°C,
vi) optional das Abspülen der Keratinfasern.

12. Verfahren zum Umformen von Keratinfasern, bevorzugt menschlichen Keratinfasern, mehr bevorzugt menschlichem Haar, umfassend die Schritte:
vii) das Auftragen einer wässrigen Zusammensetzung, die einen pH-Wert von weniger als 4 aufweist und eine oder mehr Verbindung(en) gemäß a), wie in den Ansprüchen 1 bis 10 definiert, umfasst,
viii) das Belassen der Zusammensetzung auf Keratinfasern für einen Zeitraum von 1 bis 60 min,
ix) optional das Abspülen und optional das Trocknen der Keratinfasern,
x) das Behandeln der Keratinfasern mit einem heißen Werkzeug in einem Temperaturbereich von 45°C bis 230°C,
xi) das Auftragen einer Zusammensetzung, umfassend Verbindungen b) bis d), wie in den Ansprüchen 1 bis 10 definiert, auf Keratinfasern,
xii) das Belassen der Zusammensetzung für einen Zeitraum von 1 bis 60 min,
xiii) optional das Behandeln der Keratinfasern mit einem heißen Werkzeug in einem Temperaturbereich von 45°C bis 230°C,
xiv) optional das Abspülen der Keratinfasern.

13. Verfahren gemäß Anspruch 11 und/oder 12, **dadurch gekennzeichnet, dass** der/die Glättungsschritt(e) unter Verwendung eines Glätteisens mit einer Temperatur im Bereich von 90°C bis 230°C oder unter Verwendung eines Föhns und eines Kamms im Temperaturbereich von 45°C bis 90°C durchgeführt werden.

14. Teilesatz, umfassend eine Zusammensetzung, wie in den Ansprüchen 1 bis 10 definiert, und ein Heißwerkzeug.

15. Teilesatz, umfassend eine erste Zusammensetzung, wie in Anspruch 12 Schritt vii) definiert, und eine zweite Zusammensetzung, wie in Anspruch 12 Schritt xi) definiert.

## Revendications

1. Composition cosmétique aqueuse présentant un pH de moins de 4 comprenant :
a) de l'acide glyoxylique et/ou son(ses) sel(s) et/ou son(ses)hydrate(s),
b) un ou plusieurs éthers glycéryliques d'alkyle et/ou éthers polyglycéryliques d'alkyle, et/ou leurs mélanges, dans laquelle la chaîne alkyle est une chaîne alkyle droite ou ramifiée, saturée ou insaturée présentant un nombre total de carbones de 8 ou plus,
c) un ou plusieurs composés lipophiliques,
d) un ou plusieurs tensioactifs différents du groupe b),
dans laquelle la composition est une émulsion.

2. Composition selon la revendication 1 **caractérisée en ce que** le pH de la composition est dans la plage de 0,5 à 3,5, préférablement dans la plage de 1 à 3, plus préférablement dans la plage de 1,5 à 2,5.

3. Composition selon les revendications 1 et/ou 2 **caractérisée en ce que** la concentration en composé(s) selon a) est dans la plage de 0,1 à 25 % en poids, préférablement dans la plage de 0,2 % à 20 % en poids, plus préférablement dans la plage de 0,5 % à 15 % en poids, encore plus préférablement de 1 % à 12 % en poids, calculée par rapport au poids total de la composition.

4. Composition selon l'une quelconque des revendications précédentes **caractérisée en ce que** la chaîne alkyle de composés selon b) est une chaîne alkyle droite ou ramifiée, saturée ou insaturée présentant un nombre total de carbones de 9 ou plus.

5. Composition selon l'une quelconque des revendications précédentes **caractérisée en ce qu'**un ou plusieurs composé(s) selon b) est(sont) de l'éther glycérylique d'isostéaryle.

6. Composition selon l'une quelconque des revendications précédentes **caractérisée en ce que** la concentration totale d'un ou plusieurs composé(s) selon b) est dans la plage de 0,1 % à 10 % en poids, préférablement de 0,25 % à 8 % en poids, plus préférablement de 0,5 % à 5 % en poids, calculée par rapport au poids total de la composition.

7. Composition selon l'une quelconque des revendications précédentes **caractérisée en ce que** le(s) composé(s) selon c) est(sont) sélectionné(s) parmi des alcools gras présentant une chaîne alkyle linéaire ou ramifiée, saturée ou insaturée avec un nombre total de carbones dans la plage de C8 à C22 qui peuvent être facultativement substitués par OH, des esters d'acide gras ayant estérifié un alcool linéaire ou ramifié présentant une longueur de chaîne alkyle de C3 à C22 avec un acide gras linéaire ou ramifié présentant une longueur de chaîne alkyle de C12 à C22, un(des) composé(s) à base de gelée de pétrole, un(des) organopolysiloxane(s), des huiles végétales, et/ou leurs mélanges.

8. Composition selon l'une quelconque des revendications précédentes **caractérisée en ce que** la concentration totale de composé(s) selon c) est dans la plage de 0,5 % à 25 % en poids, préférablement de 1 % à 20 % en poids, plus préférablement de 2 % à 15 % en poids, calculée par rapport au poids total de la composition.

9. Composition selon l'une quelconque des revendications précédentes **caractérisée en ce qu'**un ou plusieurs composé(s) selon d) est(sont) sélectionné(s) parmi un(des) tensioactif(s) non ionique(s), un(des) tensioactif(s) cationique(s), un(des) tensioactif(s) anionique(s), un(des) tensioactif(s) amphotère(s)/zwittérionique(s), et/ou leurs mélanges, étant préférablement sélectionné(s) parmi un(des) tensioactif(s) non ionique(s) et/ou cationique(s).

10. Composition selon l'une quelconque des revendications précédentes **caractérisée en ce que** la concentration totale de composé(s) selon d) est dans la plage de 0,2 % à 10 % en poids, préférablement de 0,5 % à 5 % en poids, plus préférablement de 1 % à 3 % en poids, calculée par rapport au poids total de la composition.

11. Procédé de remise en forme de fibres kératiniques, préférablement de fibres kératiniques humaines, plus préférablement de cheveux humains, comprenant les étapes consistant à :
i) appliquer la composition telle que définie dans les revendications 1 à 10 sur des fibres kératiniques et la laisser pendant une durée allant de 1 à 60 min,
ii) rincer facultativement les fibres kératiniques et sécher facultativement les fibres kératiniques,
iii) traiter les fibres kératiniques à l'aide d'un accessoire chaud dans une plage de températures de 45 °C à 230 °C,
iv) rincer facultativement les fibres kératiniques,
v) boucler facultativement les fibres kératiniques à l'aide d'un accessoire à boucler dans une plage de températures de 45 °C à 230 °C,
vi) rincer facultativement les fibres kératiniques.

12. Procédé de remise en forme de fibres kératiniques, préférablement de fibres kératiniques humaines, plus préférablement de cheveux humains, comprenant les étapes consistant à :
vii) appliquer une composition aqueuse présentant un pH de moins de 4 et comprenant un ou plusieurs composé(s) selon a) telle que définie dans les revendications 1 à 10,
viii) laisser la composition sur les fibres kératiniques pendant une durée de 1 à 60 min,
ix) rincer facultativement et sécher facultativement les fibres kératiniques,
x) traiter les fibres kératiniques à l'aide d'un accessoire chaud dans une plage de températures de 45 °C à 230 °C,
xi) appliquer une composition comprenant des composés b) à d) telle que définie dans les revendications 1 à 10 sur les fibres kératiniques,
xii) laisser la composition pendant une durée de 1 à 60 min,
xiii) traiter facultativement les fibres kératiniques à l'aide d'un accessoire chaud dans une plage de températures de 45 °C à 230 °C,
xiv) rincer facultativement les fibres kératiniques.

13. Procédé selon la revendication 11 et/ou 12 **caractérisé en ce que** la(les) étape(s) de lissage est(sont) mise(s) en œuvre en utilisant un fer à lisser présentant une température dans la plage de 90 °C à 230 °C ou en utilisant un sèche-cheveux et un peigne dans la plage de températures de 45 °C à 90 °C.

14. Kit de pièces comprenant une composition telle que définie dans les revendications 1 à 10 et un accessoire chaud.

15. Kit de pièces comprenant une première composition telle que définie à l'étape vii) de la revendication 12 et une seconde composition telle que définie dans l'étape xi) de la revendication 12.
